# FASCICULE DE BREVET EUROPEEN

(11) **EP 4 387 707 B1**
(45) Date de publication et mention de la délivrance du brevet: **22.10.2025**
(21) Numéro de dépôt: 22826818.1
(22) Date de dépôt: 01.12.2022
(51) Int. Cl.: A61M 60/174, A61M 60/237, A61M 60/422, A61M 60/806, A61M 60/81, A61M 60/812

(54) **POMPE CARDIAQUE INTRAVENTRICULAIRE À TÊTE RÉTRÉCIE**
INTRAVENTRIKULÄRE HERZPUMPE MIT VERENGTEM KOPF
INTRAVENTRICULAR CARDIAC PUMP WITH NARROWED HEAD

(30) Priorité: 21.12.2021 FR 2114112
(43) Date de publication de la demande: 26.06.2024
(73) Titulaire: Fineheart, 33600 Pessac (FR)
(72) Inventeur: GARRIGUE, Stéphane, 33130 BEGLES (FR); MASCARELL, Arnaud, 37250 MONTBAZON (FR)
(74) Mandataire: IPAZ
(86) Numéro de dépôt international: PCT/EP2022/084129
(87) Numéro de publication internationale: WO 2023/117368

(56) Documents cités:
- CA-A1- 2 472 088
- CA-A1- 2 517 236
- US-A1- 2021 260 359

## Description

La présente invention se rapporte à une pompe, notamment axiale, destinée à être immergée dans un fluide.

La présente invention concerne une pompe pour assistance ventriculaire. Il s'agit par exemple d'une pompe alimentée par batterie et destinée à être insérée dans un corps humain pour aider à la circulation du sang.

L'insuffisance cardiaque (IC), incapacité progressive du cœur à fournir un débit sanguin nécessaire aux besoins métaboliques d'un individu dans la vie courante, est la seconde cause de mortalité dans les pays occidentaux. Le traitement de l'insuffisance cardiaque, qui consiste à augmenter le débit sanguin de façon adaptée aux besoins du patient, progresse mais reste encore insuffisant.

Une pompe cardiaque intraventriculaire est destinée à être insérée dans un ventricule systémique du cœur d'un patient. Tous les cœurs ne présentent pas des dimensions identiques. Il pourrait être nécessaire de concevoir plusieurs pompes de tailles différentes.

On connaît le document CA2472088A1 décrivant une pompe cardiaque de type à la fois radial et axial. Ce document décrit un caisson doté d'une ouverture latérale pour l'entrée de sang. Une turbine interne permet de propulser le sang vers une tête de sortie supérieure.

La présente invention a pour but d'éviter de telles conceptions en proposant une nouvelle pompe adaptée pour être compatible à de nombreuses tailles de cœur.

L'invention a aussi pour but une pompe efficace quel que soit l'état du cœur, dilaté ou pas.

On atteint au moins l'un des objectifs avec une pompe cardiaque selon la revendication 1.

Par section, on entend l'aire de la surface vue selon une coupe perpendiculaire à l'axe de propulsion du fluide. Par section externe, on entend une section dont le contour épouse le contour extérieur de la chambre de propulsion, au contraire d'une section interne qui concernerait le volume interne dans lequel le fluide circule.

La pompe selon la présente invention a pour avantage de présenter une tête rétrécie dont la dimension est adaptée pour propulser efficacement le fluide vers les valves sigmoïdes à l'entrée de l'aorte. La plage de 14 à 16mm permet d'avoir une tête de sortie convenable quel que soit l'état du cœur. En effet, que le cœur soit dilaté ou pas, qu'il s'agisse d'un cœur d'enfant ou d'un cœur d'adulte c'est-à-dire un petit cœur ou un gros cœur, la plage définie par la présente invention permet de positionner efficacement la pompe proche de l'aorte sans entrer en contact. Par proche on entend une distance suffisante pour que le flux de fluide propulsé soit suffisamment puissant pour écarter les valves sigmoïdes et pour que le fluide pénètre dans l'aorte. Cette distance dépend de la puissance de la pompe.

Un même cœur peut présenter différentes dimensions. Par exemple, un cœur dilaté présente un volume largement supérieur au volume du même cœur en situation non-dilatée. De manière remarquable, la dimension de l'entrée de l'aorte reste identique quel que soit le niveau de dilation du cœur.

La dimension de la tête permet également le passage d'un flux naturel ou spontané. Dans le cadre d'une insuffisance cardiaque, le cœur peut encore être capable de se contracter pour repousser, insuffisamment, le fluide vers l'aorte. Le flux créé est ainsi un flux naturel alors que le flux généré par la pompe est un flux induit qui vient en complément majoritaire ou en remplacement du flux naturel.

De ce fait, proposer une dimension fixe pour la tête de sortie comprise entre 14mm et 16mm selon l'invention, permet une bonne circulation des flux induit et naturel. Cette dimension autorise un passage suffisant du flux naturel autour de la tête.

Selon une caractéristique avantageuse de l'invention, la section externe du corps supérieur peut présenter un diamètre, ou une plus grande distance, compris entre 17 et 20mm.

Selon un mode de réalisation de l'invention, la section externe du corps supérieur présente un diamètre, ou une plus grande distance, égal à 18mm.

Avec un tel arrangement, le corps supérieur est convenablement dimensionné pour accueillir des moyens pour propulser le fluide vers la tête de sortie. Le corps supérieur est plus large que la tête de sortie.

Avantageusement, la tête de sortie peut présenter une distance le long d'un axe de propulsion compris entre 5 et 10mm. Une telle tête peut par exemple être de la forme d'un cylindre droit guidant le flux de fluide vers l'extérieur en direction de l'aorte.

La tête de sortie peut par exemple comporter des pales, l'ensemble des pales constituant un redresseur apte à augmenter la vitesse et donner au fluide un profil prédéterminé en sortie.

Selon une caractéristique complémentaire, la pompe selon l'invention peut comprendre un épaulement à la liaison entre le corps supérieur et la tête de sortie, cet épaulement présentant une forme concave. Par forme concave on entend une forme arrondie vers l'intérieur de façon à ce que le flux naturel coule le long de l'épaulement sans stagnation.

Selon une caractéristique avantageuse de l'invention, la chambre de propulsion peut être en forme de cylindre circulaire droit.

Selon un mode de réalisation avantageux, le corps supérieur peut présenter une forme externe évasée depuis la chambre d'admission jusqu'à la tête de sortie. De préférence, la progression du diamètre de la chambre de propulsion est progressive et sans épaulement.

Le diamètre de la tête peut être inférieur, supérieur ou égal au plus petit diamètre de la chambre de propulsion..

Selon une caractéristique avantageuse de l'invention, les éléments de liaison peuvent présenter une forme générale galbée vers l'extérieur. La forme galbée peut signifier que les éléments de liaisons s'inscrivent le long d'une forme arrondie, globalement sphérique, dont le diamètre est supérieur au diamètre de l'extrémité inférieure de la chambre de propulsion et au diamètre de l'extrémité supérieur de la partie inférieure du carter.

A titre d'exemple, les éléments de liaison peuvent comprendre quatre tiges.

Selon une caractéristique avantageuse de l'invention, les tiges peuvent comporter des prolongements jusqu'à l'intérieur de la chambre de propulsion ; ces prolongements servant de pales d'inducteur (« inducer » en anglais) pour rendre l'écoulement du fluide linéaire en direction de la partie supérieure du carter.

Par exemple, les tiges peuvent être reliées entre elles par une bande latérale disposée à distance des extrémités de ces tiges.

Selon un mode de mise en œuvre de l'invention, la chambre de propulsion peut comprendre un rotor pour propulser le fluide vers la tête de sortie.

Avantageusement, la pompe peut comprendre une unité de traitement configurée pour contrôler le rotor selon un mode pulsé.

D'autres avantages et caractéristiques de l'invention apparaîtront à l'examen de la description détaillée d'un mode de mise en œuvre nullement limitatif, et des dessins annexés, sur lesquels :
[Fig. 1] La figure 1 est une vue générale d'une pompe cardiaque intraventriculaire selon l'invention,
[Fig. 2] La figure 2 est une en perspective d'un carter d'une pompe cardiaque intraventriculaire selon l'invention,
[Fig. 3] La figure 3 est une vue schématique simplifiée illustrant les dimensions d'une tête rétrécie selon l'invention,
[Fig. 4] La figure 4 est une vue schématique d'une turbine à pales hélicoïdales à l'extérieur du rotor selon l'invention,
[Fig. 5] La figure 5 est une vue schématique simplifiée illustrant les prolongements de tiges formant pales inducteur et une turbine à pales hélicoïdales internes selon l'invention,
[Fig. 6] La figure 6 est une vue schématique d'une turbine à pales hélicoïdales internes selon l'invention.

Les modes de réalisation qui seront décrits dans la suite ne sont nullement limitatifs; on pourra notamment mettre en œuvre des variantes de l'invention ne comprenant qu'une sélection de caractéristiques décrites par la suite isolées des autres caractéristiques décrites, si cette sélection de caractéristiques est suffisante pour conférer un avantage technique ou pour différencier l'invention par rapport à l'état de la technique antérieur. Cette sélection comprend au moins une caractéristique de préférence fonctionnelle sans détails structurels, ou avec seulement une partie des détails structurels si cette partie uniquement est suffisante pour conférer un avantage technique ou pour différencier l'invention par rapport à l'état de la technique antérieur.

En particulier toutes les variantes et tous les modes de réalisation décrits sont prévus pour être combinés entre eux dans toutes les combinaisons où rien ne s'y oppose sur le plan technique.

Sur les figures, les éléments communs à plusieurs figures conservent la même référence.

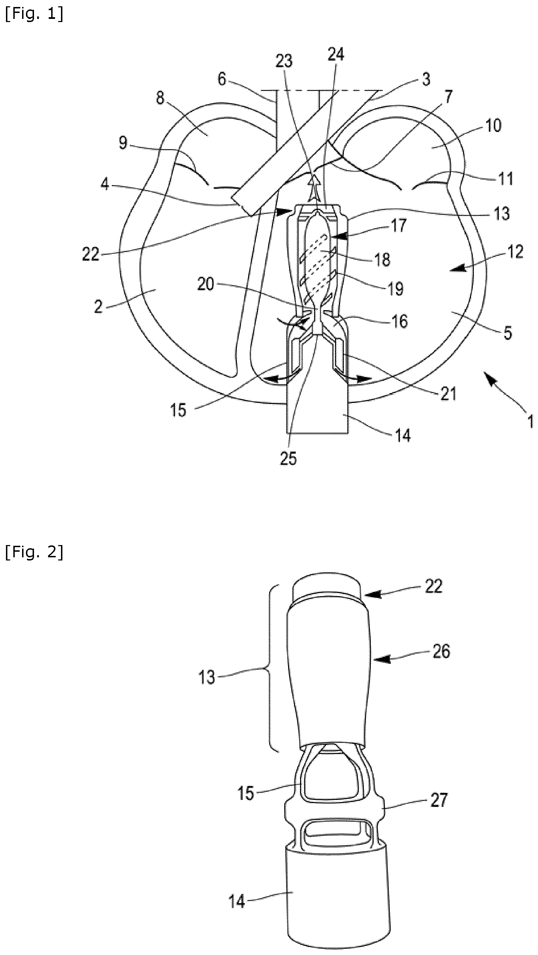

Sur la figure 1 est illustrée une pompe cardiaque intraventriculaire.

Le cœur est globalement désigné par la référence 1. On distingue le ventricule droit 2 qui a pour fonction d'éjecter le sang vers l'artère pulmonaire 3 à travers des valves sigmoïdes 4. Le ventricule gauche 5 a pour fonction de réaliser la circulation systémique en éjectant le sang rempli d'oxygène vers l'aorte 6 via des valves sigmoïdes 7.

L'oreillette droite 8 alimente le ventricule droit 2 en sang via des valves auriculo-pulmonaires 9. L'oreillette gauche 10 alimente le ventricule gauche 5 en sang via les valves auriculo-pulmonaires 11.

La pompe selon l'invention est globalement référencée en 12. Elle est fixée à l'apex du ventricule gauche 5. Elle peut être connectée de façon filaire ou sans fil à une unité de gestion (non représentée) externe au cœur. Elle peut être connectée à une ou plusieurs sondes ou capteurs (non représentés) pour la détection du rythme cardiaque ou autre.

La partie inférieure de la pompe logeant le moteur peut être partiellement à l'extérieur du cœur, partiellement dans l'épaisseur de l'apex ou entièrement dans le cœur. Dans l'exemple de la figure 1, la partie inférieure de la pompe est partiellement à l'extérieure du cœur, ce qui peut être un avantage pour la maintenance du moteur notamment.

La pompe comprend un carter composé d'une partie supérieure 13 reliée de façon rigide à une partie inférieure 14 au moyen d'éléments de liaison 15. Ces éléments de liaison peuvent comprendre une ou plusieurs tiges 15 reliant les deux parties 13 et 14 tout en laissant de larges passages pour le sang.

Dans l'exemple de la figure 1, la partie inférieure 14 constitue le stator d'un moteur. Le carter, 13, 14 et 15, est destiné à rester fixe. Entre la partie supérieure 13 et la partie inférieure 14, se trouve une chambre d'admission 16 qui est un espace ouvert, seulement entravé par les éléments de liaison 15. En fonctionnement, la pompe est prévue pour aspirer le sang contenu dans le ventricule 5 via la chambre d'admission 16, le conduire à travers la partie supérieure 13 du carter et l'éjecter par la sortie supérieure vers la valve 7.

Pour aspirer le sang, la pompe comprend une turbine (« impeller » en anglais) 17 conçue à partir d'un corps oblongue 18 autour duquel est enroulée une ou plusieurs pales hélicoïdales 19. En rotation, la turbine aspire le sang et le propulse vers la sortie. Il s'agit de la fonction principale de la pompe. Le flux principal est donc celui qui est pompé par la turbine 17.

La turbine est portée par un arbre de transmission 20 qui comporte à l'extrémité opposée de la turbine une cloche 21. L'ensemble turbine 17, arbre de transmission 20 et cloche 21 est rigide et apte à effectuer des rotations. Pour ce faire, la cloche 21 constitue le rotor du moteur formé avec le stator fixe 14. Ce moteur 14 et 21 est un moteur de type « brushless » à rotor externe. Il s'agit d'une machine synchrone dotée d'un système électronique de commande (non représenté) accessible de l'extérieur du cœur ou non.

La turbine est apte à réaliser des mouvements de rotation selon son axe et relativement au carter 13, 14 qui reste fixe. Dans l'axe de rotation de la turbine se trouve un pivot d'entrée 25 et un pivot de sortie 23 qui maintiennent la turbine dans son axe lorsque celle-ci est en sustentation magnétique lors des mouvements de rotation. Dans une variante non représentée, l'une ou les deux zones de pivot peuvent comprendre des liaisons à roulements autorisant la rotation de la turbine.

L'invention est notamment remarquable par le fait que la partie supérieure 13 du carter comprend une tête de sortie 22 pour propulser le sang à l'extérieur de la pompe, en direction de l'aorte 6.

Sur la figure 2 est illustrée en perspective une pompe selon l'invention. On distingue la partie inférieure 14, les tiges 15 et la partie supérieure 13. Cette dernière comprend un corps supérieur 26 et la tête de sortie 22. Le corps supérieur 26 présente une forme évasée, les dimensions augmentant depuis les tiges 15 jusqu'à la tête de sortie 22. Le corps supérieur 26 se termine par un épaulement venant réduire son diamètre pour connecter la tête de sortie qui présente un diamètre inférieur au diamètre de la partie haute du corps supérieur (lorsque la pompe est disposée selon la verticale, la partie inférieure étant vers le bas).

Il est également prévu une bande latérale 27 reliant les tiges entre elles de façon à solidifier les tiges de la chambre d'admission, tout en laissant des ouvertures pour l'admission du sang et l'évacuation d'un sang de rétrodiffusion entre la cloche 21 et la partie inférieure fixe 14.

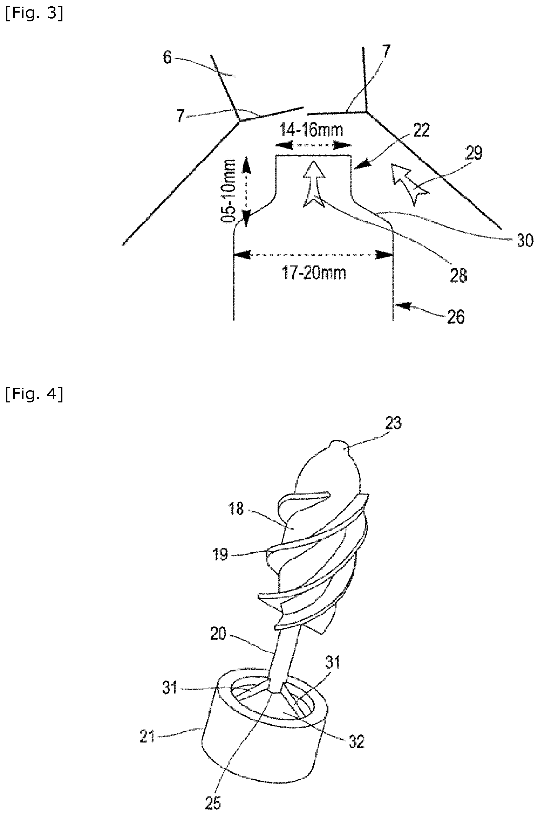

La figure 3 est une vue schématique illustrant quelques dimensions de la tête de sortie et du corps supérieur.

La présente invention impose de fixer le diamètre de la tête de sortie entre 14 et 16mm. Cette plage de valeurs permet de faire cohabiter à la fois un flux induit 28 provenant de la pompe et un flux spontané ou naturel 29 provenant de la contraction naturelle du cœur. En effet, ce dimensionnement est compatible avec toute dimension de cœur et quel que soit l'état du cœur, c'est-à-dire dilaté ou non. Le rétrécissement de la tête permet un positionnement efficace de la pompe, proche de l'aorte sans toutefois bloquer le passage du flux spontané. Le diamètre de la tête est suffisamment large pour pouvoir générer un flux capable d'atteindre l'aorte.

De préférence, la hauteur de la tête de sortie est comprise entre 5 et 10mm. Un coude 30 permet de faire la liaison entre la tête de sortie 22 de diamètre réduit et le corps supérieur 26 de diamètre supérieur au diamètre de la tête de sortie. Le corps supérieur présente sur sa partie supérieure un diamètre compris entre 17 et 20mm. On prévoit notamment, lorsque le diamètre du corps supérieur est supérieur à 17mm, le diamètre de la tête de sortie peut atteindre 17mm.

Sur la figure 4 est représentée un peu plus en détail la turbine 17 reliée à l'arbre de transmission 20 et à la cloche 21. Le pivot de sortie 23, en forme de demi-sphère, se trouve au sommet de la turbine et dans l'axe de rotation. Les pales hélicoïdales 19 entourent le corps 18 depuis le pied jusqu'à couvrir environ les trois-quarts du corps 18. Le sommet de la turbine est dépourvu de pales.

L'arbre de transmission 20 est solidement connecté à des pales latérales 31 dont deux sont visibles et une troisième cachée. Les pales latérales sont au nombre de trois, mais il peut y en avoir qu'une, deux ou plus de trois. Dans tous les cas, les pales latérales 31 doivent laisser des ouvertures 32 pour que le fluide puisse pénétrer à l'intérieur de la cloche 21. Dans l'exemple illustré, le pivot d'entrée 25 se trouve au-dessus de la cloche 21, mais on peut envisager d'autres modes de réalisation où le pivot d'entrée est disposé à l'intérieur de la cloche 21.

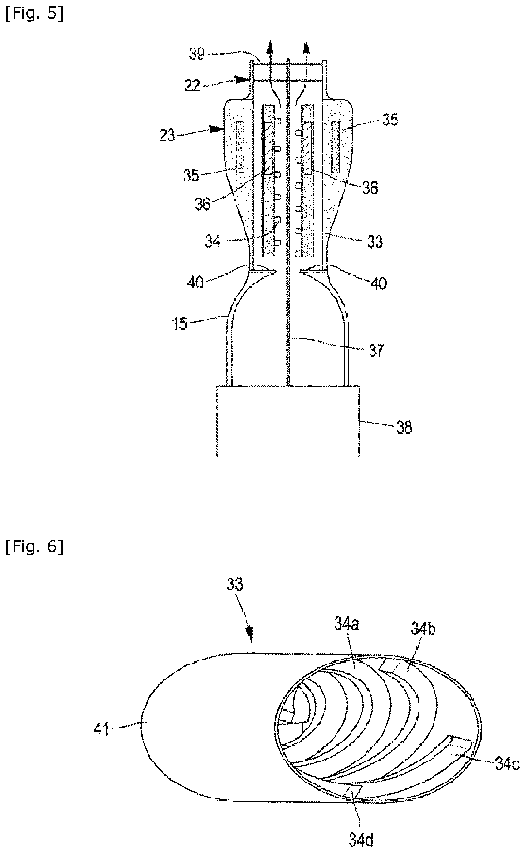

Sur la figure 5 est représentée, en vue de coupe, une turbine à pales hélicoïdales internes. Le moteur de la pompe comprend un stator fixe, qui est le corps supérieur 23, et un rotor ou turbine 33. Dans l'exemple de la figure 5, la turbine 33 est un cylindre droit creux. La partie creuse présente également une forme cylindrique droit, mais elle peut présenter une toute autre forme telle qu'une forme évasée, oblongue ou structurée. La partie centrale creuse de la turbine 33 porte sur sa surface interne une pale 34 hélicoïdale pour propulser le fluide vers la tête de sortie 22. Il peut s'agit d'une seule pale 34 ou plusieurs pales, à pas fixe ou variable, sur toute la longueur de la surface interne de la turbine ou sur une partie seulement. La turbine 33 est conçue pour être en rotation par rapport au corps supérieur 23. Idéalement le corps supérieur 23 est un stator comportant des éléments magnétiques tels que des enroulements magnétiques 35. Ces derniers peuvent coopérer magnétiquement avec des éléments magnétiques tels que par exemple des aimants permanents 36 disposés à l'intérieur ou sur la surface externe de la turbine 33. Des moyens électroniques (non représentés) sont prévus pour commander les enroulements 35 de façon à mettre en action la turbine 33. L'ensemble constitue un moteur de type « brushless ». L'entrefer entre la turbine 33 et le corps supérieur 23 est droit, il s'inscrit dans un cylindre droit, mais il peut être d'une autre forme non droite, évasée ou non.

De façon optionnelle, une colonne fixe 37 est disposée à l'intérieur de la turbine 33 et permet d'améliorer l'écoulement du sang lorsque la pompe est en fonctionnement, c'est-à-dire la turbine 33 en rotation par rapport au corps supérieur 23. Cette colonne 37 peut être fixée par les deux extrémités, par exemple à une partie inférieure fixe 38 du carter et à la tête de sortie 22 via des pales de sortie 39. On peut également envisager une colonne 37 fixée à une seule extrémité.

D'autres modes de disposition de la colonne 37 peuvent être envisagées, comme par exemple une colonne en rotation mais fixe en translation, dans ce cas la colonne 37 peut être fixée à la turbine 33 au moyen d'un bras permettant à la colonne de rester dans l'axe de rotation de la turbine.

Des mécanismes à roulement peuvent être prévus entre la turbine et le corps supérieur 23 pour maintenir la turbine. D'autres moyens peuvent être prévus pour maintenir la turbine en rotation dans le carter sans contact. On peut notamment envisager un mécanisme par sustentation, par fluide, par aimantation,..., avec ou sans des rebords de maintien.

Sur la figure 5, on distingue également des prolongements 40 des tiges 15. Ces prolongements constituent des pales d'inducteur à l'intérieur de la chambre de propulsion. Ces prolongements sont disposés en entrée de la chambre de propulsion 23, 22 pour rendre l'écoulement du fluide linéaire en direction de la turbine.

Sur la figure 6 est représenté un exemple de turbine 33 de la figure 5. Il s'agit, dans l'exemple décrit de façon non limitative, d'un corps 41 en forme de cylindre creux longiligne dont la paroi interne comporte plusieurs pales 34a, 34b, 34C, 34d. Une telle turbine peut avantageusement être utilisée dans une pompe plongée dans un fluide.

Les pales 34a, 34b, 34C, 34d ont pour fonction de faire transiter le fluide à travers la turbine. L'orientation et le dimensionnement des pales sont prévus pour que le fluide soit aspiré puis propulsé après avoir traversé la turbine qui serait en rotation.

Dans l'exemple de la figure 6, les quatre pales hélicoïdales 34a, 34b, 34C, 34d, partent d'une extrémité de la turbine, s'inscrivent dans des lignes hélicoïdales sans jamais se croiser, et arrivent à l'autre extrémité.

Bien entendu, l'invention n'est pas limitée aux exemples qui viennent d'être décrits. De nombreuses modifications peuvent être apportées à ces exemples sans sortir du cadre de la présente invention qui est défini par les revendications suivantes.

## Revendications

1. Pompe cardiaque (12) destinée à être positionnée partiellement ou entièrement dans un ventricule d'un cœur (1) et à générer un flux induit de fluide en direction de valves sigmoïdes de l'aorte ; cette pompe comprenant :
- un carter fixe (13, 14, 15) doté, lorsque la pompe cardiaque est positionnée verticalement, d'une partie supérieure (13) formant chambre de propulsion pour propulser le fluide vers l'extrémité supérieure, et une partie inférieure (14) reliée de manière fixe à la partie supérieure,
- au moins une ouverture latérale entre la partie supérieure et la partie inférieure, et formant une chambre d'admission (16) du fluide depuis l'extérieur vers la chambre de propulsion,
- un bloc moteur (14, 21) disposé à l'intérieur du carter pour entraîner le fluide depuis l'ouverture latérale jusqu'à la sortie de la chambre de propulsion,
- la chambre de propulsion comprend un corps supérieur (26) et une tête de sortie (22) ; la tête de sortie présentant une section externe rétrécie par rapport à une section externe du corps supérieur ; la tête de sortie (22) présentant une section externe dont le diamètre, ou la plus grande distance, est compris entre 14 et 16mm ; et en ce que la chambre de propulsion (13) est reliée de manière fixe à la partie inférieure du carter au moyen d'éléments de liaison (15) dont la partie supérieure est directement connectée à l'extrémité inférieure de la chambre de propulsion (13), la pompe cardiaque étant **caractérisé en ce que** la surface externe de la chambre de propulsion affleure les éléments de liaison à l'endroit de connexion.

2. Pompe cardiaque selon la revendication 1, **caractérisé en ce que** la section externe du corps supérieur (26) présente un diamètre, ou une plus grande distance, compris entre 17 et 20mm.

3. Pompe cardiaque selon la revendication 1 ou 2, **caractérisé en ce que** la section externe du corps supérieur (26) présente un diamètre, ou une plus grande distance, égal à 18mm.

4. Pompe cardiaque selon l'une quelconque des revendications précédentes, **caractérisé en ce que** la tête de sortie (22) présente une distance le long d'un axe de propulsion compris entre 5 et 10mm.

5. Pompe cardiaque selon l'une quelconque des revendications précédentes, **caractérisé en ce qu'**elle comprend un épaulement (30) à la liaison entre le corps supérieur et la tête de sortie, cet épaulement présentant une forme concave.

6. Pompe cardiaque selon l'une quelconque des revendications précédentes, **caractérisé en ce que** la chambre de propulsion (13) est en forme de cylindre circulaire droit.

7. Pompe cardiaque selon l'une quelconque des revendications précédentes, **caractérisé en ce que** le corps supérieur (26) présente une forme externe évasée depuis la chambre d'admission jusqu'à la tête de sortie.

8. Pompe cardiaque selon l'une quelconque des revendications précédentes, **caractérisé en ce que** les éléments de liaison (15) présentent une forme générale galbée vers l'extérieur.

9. Pompe cardiaque selon l'une quelconque des revendications précédentes, **caractérisé en ce que** les éléments de liaison (15) comprennent quatre tiges.

10. Pompe cardiaque selon la revendication 9, **caractérisé en ce que** les tiges comportent des prolongements (40) jusqu'à l'intérieur de la chambre de propulsion (13) ; ces prolongements servant de pales d'inducteur pour rendre l'écoulement du fluide linéaire en direction de la partie supérieure du carter.

11. Pompe cardiaque selon l'une quelconque des revendications 9 ou 10, **caractérisé en ce que** les tiges sont reliées entre elles par une bande latérale (27) disposée à distance des extrémités.

12. Pompe cardiaque selon l'une quelconque des revendications précédentes, **caractérisé en ce que** la chambre de propulsion comprend un rotor (33) pour propulser le fluide vers la tête de sortie.

13. Pompe cardiaque selon la revendication 12, **caractérisé en ce que** qu'elle comprend une unité de traitement configurée pour contrôler le rotor selon un mode pulsé.

## Patentansprüche

1. Herzpumpe (12), die dazu bestimmt ist, teilweise oder vollständig in einem Ventrikel eines Herzens (1) positioniert zu werden und einen induzierten Fluidstrom in Richtung der Taschenklappen der Aorta zu erzeugen; wobei diese Pumpe umfasst:
- ein festes Gehäuse (13, 14, 15), das bei vertikaler Positionierung der Herzpumpe einen oberen Teil (13), der eine Antriebskammer zum Vortreiben des Fluids zum oberen Ende bildet, und einen unteren Teil (14), der fest mit dem oberen Teil verbunden ist, aufweist,
- mindestens eine seitliche Öffnung zwischen dem oberen Teil und dem unteren Teil, die eine Einlasskammer (16) für das Fluid von außen in Richtung der Antriebskammer bildet,
- eine im Gehäuse angeordnete Motoreinheit (14, 21), um das Fluid von der seitlichen Öffnung zum Auslass der Antriebskammer zu befördern,
- wobei die Antriebskammer einen oberen Körper (26) und einen Auslasskopf (22) umfasst; wobei der Auslasskopf einen im Vergleich zu einem Außenquerschnitt des oberen Körpers verengten Außenquerschnitt aufweist; wobei der Auslasskopf (22) einen Außenquerschnitt aufweist, dessen Durchmesser oder größte Weite zwischen 14 und 16 mm liegt; und dass die Antriebskammer (13) mittels Verbindungselementen (15), deren oberer Teil direkt mit dem unteren Ende der Antriebskammer (13) verbunden ist, fest mit dem unteren Teil des Gehäuses verbunden ist, wobei die Herzpumpe **dadurch gekennzeichnet ist, dass** die Außenfläche der Antriebskammer an der Verbindungsstelle bündig mit den Verbindungselementen ist.

2. Herzpumpe nach Anspruch 1, **dadurch gekennzeichnet, dass** der Außenquerschnitt des oberen Körpers (26) einen Durchmesser bzw. eine größte Weite zwischen 17 und 20 mm aufweist.

3. Herzpumpe nach Anspruch 1 oder 2, **dadurch gekennzeichnet, dass** der Außenquerschnitt des oberen Körpers (26) einen Durchmesser bzw. eine größte Weite von 18 mm aufweist.

4. Herzpumpe nach einem der vorhergehenden Ansprüche, **dadurch gekennzeichnet, dass** der Auslasskopf (22) eine Weite entlang einer Antriebsachse von zwischen 5 und 10 mm aufweist.

5. Herzpumpe nach einem der vorhergehenden Ansprüche, **dadurch gekennzeichnet, dass** sie an der Verbindung zwischen dem oberen Körper und dem Auslasskopf eine Schulter (30) umfasst, die eine konkave Form aufweist.

6. Herzpumpe nach einem der vorhergehenden Ansprüche, **dadurch gekennzeichnet, dass** die Antriebskammer (13) die Form eines geraden Kreiszylinders aufweist.

7. Herzpumpe nach einem der vorhergehenden Ansprüche, **dadurch gekennzeichnet, dass** der obere Körper (26) von der Einlasskammer bis zum Auslasskopf eine konisch erweiterte Außenform aufweist.

8. Herzpumpe nach einem der vorhergehenden Ansprüche, **dadurch gekennzeichnet, dass** die Verbindungselemente (15) eine im Allgemeinen nach außen gewölbte Form aufweisen.

9. Herzpumpe nach einem der vorhergehenden Ansprüche, **dadurch gekennzeichnet, dass** die Verbindungselemente (15) vier Stege umfassen.

10. Herzpumpe nach Anspruch 9, **dadurch gekennzeichnet, dass** die Stege bis in die Antriebskammer (13) hineinragende Verlängerungen (40) umfassen; wobei diese Verlängerungen als Induktorschaufeln dienen, um den Fluidstrom zum oberen Teil des Gehäuses geradlinig zu gestalten.

11. Herzpumpe nach einem der Ansprüche 9 oder 10, **dadurch gekennzeichnet, dass** die Stege durch einen im Abstand zu den Enden angeordneten Seitenstreifen (27) miteinander verbunden sind.

12. Herzpumpe nach einem der vorhergehenden Ansprüche, **dadurch gekennzeichnet, dass** die Antriebskammer einen Rotor (33) zum Vortreiben des Fluids in Richtung des Auslasskopfes umfasst.

13. Herzpumpe nach Anspruch 12, **dadurch gekennzeichnet, dass** sie eine Verarbeitungseinheit umfasst, die dazu konfiguriert ist, den Rotor in einem gepulsten Modus zu steuern.

## Claims

1. Heart pump (12) intended to be positioned partially or entirely in a ventricle of a heart (1) and to generate an induced flow of fluid in the direction of the sigmoid valves of the aorta; this pump comprising:
- a fixed casing (13, 14, 15) provided with a top part (13), when the heart pump is positioned vertically, forming a propulsion chamber for propelling the fluid towards the top end, and a bottom part (14) attached in a fixed manner to the top part,
- at least one side opening between the top part and the bottom part and forming a chamber (16) for fluid inlet from the outside towards the propulsion chamber,
- a power unit ( 14, 21) arranged inside the casing to drive the fluid from the side opening right up to the outlet of the propulsion chamber,
- the propulsion chamber comprises an upper body (26) and an outlet head (22); the outlet head having a narrowed external cross section with respect to an external cross section of the upper body; the outlet head (22) having an external cross section the diameter, or the largest distance, of which is comprised between 14 and 16 mm; and in that the propulsion chamber (13) is attached in a fixed manner to the bottom part of the casing by means of linking elements (15) the top part of which is directly connected to the bottom end of the propulsion chamber (13),
the heart pump being **characterized in that** the outer surface of the propulsion chamber is flush with the linking elements at the place of connection.

2. Heart pump according to claim 1, **characterized in that** the external cross section of the upper body (26) has a diameter, or a largest distance, comprised between 17 and 20 mm.

3. Heart pump according to claim 1 or 2, **characterized in that** the external cross section of the upper body (26) has a diameter, or a largest distance, equal to 18 mm.

4. Heart pump according to any one of the preceding claims, **characterized in that** the outlet head (22) has a distance along a propulsion axis comprised between 5 and 10 mm.

5. Heart pump according to any one of the preceding claims, **characterized in that** it comprises a shoulder (30) at the connection between the upper body and the outlet head, this shoulder having a concave shape.

6. Heart pump according to any one of the preceding claims, **characterized in that** the propulsion chamber (13) is in the shape of a straight circular cylinder.

7. Heart pump according to any one of the preceding claims, **characterized in that** the upper body (26) has an outer shape that is flared from the inlet chamber right up to the outlet head.

8. Heart pump according to any one of the preceding claims, **characterized in that** the linking elements (15) have a general shape that is curved toward the outside.

9. Heart pump according to any one of the preceding claims, **characterized in that** the linking elements (15) comprise four pillars.

10. Heart pump according to claim 9, **characterized in that** the pillars include prolongations (40) right up to the inside of the propulsion chamber (13); these prolongations serving as inducer vanes to make the flow of the fluid linear in the direction of the top part of the casing.

11. Heart pump according to any one of claims 9 or 10, **characterized in that** the pillars are attached to one another by a lateral (27) band arranged at a distance from the ends.

12. Heart pump according to any one of the preceding claims, **characterized in that** the propulsion chamber comprises a rotor (33) to propel the fluid towards the outlet head.

13. Heart pump according to claim 12, **characterized in that** it comprises a processing unit configured to control the rotor according to a pulsed mode.
